# EUROPEAN PATENT SPECIFICATION

(11) **EP 1 740 065 B1**
(45) Date of publication and mention of the grant of the patent: **08.09.2010**
(21) Application number: 05729034.8
(22) Date of filing: 24.03.2005
(51) Int. Cl.: A42B 1/24

(54) **CAP EQUIPPED WITH LIGHT**
MÜTZE MIT EINEM LICHT
CASQUETTE EQUIPE D'UNE LUMIERE

(30) Priority: 05.04.2004 FI 20040487; 11.08.2004 FI 20041072
(43) Date of publication of application: 10.01.2007
(73) Proprietor: Sky Crest Europe Ltd., AX-22101 Mariehamn, Åland (FI)
(72) Inventor: KARLSSON, Ralf, FI-22100 Maarianhamina (FI)
(74) Representative: Kupiainen, Juhani Kalervo
(86) International application number: PCT/FI2005/000164
(87) International publication number: WO 2005/096856

(56) References cited:
- US-A- 5 485 358
- US-A- 5 510 961
- US-A- 5 667 292
- US-A- 5 680 718
- US-A- 5 758 947
- US-A- 6 056 413
- US-A1- 2003 117 575
- US-A1- 2003 231 489
- US-A1- 2004 264 173
- US-B1- 6 168 286
- US-B1- 6 733 150

## Description

### OBJECT OF THE INVENTION

The object of the present invention is a cap with an illuminator. Combining a cap with an illuminator conveniently produces an illuminator that one can easily carry anywhere for possible use. This makes it unnecessary to have a separate illuminator. Perhaps the greatest advantage, however, of an illuminator combined with a cap is that the hands remain free for work. The device thus conveniently replaces a separate forehead lamp held in place by straps also in the respect that a forehead lamp is usually worn much less often than a cap.

### PRIOR ART

It is not unknown to connect an illuminator to an item of headgear, but such illuminators are large in size and cumbersome. The headgear may not be worn as items of headgear are usually worn because the illuminator is in the way when not in use.

Document US-A-2003/0 117 575 discloses a cap with LED lights on its brim. Stacked batteries are located in a housing on the brim or at the back of the cap.

### OBJECT OF THE INVENTION

The purpose of this invention is to create a device, which overcomes the above-mentioned drawbacks.

### CHARACTERISTICS OF THE INVENTION

An illuminator according to the invention is characterised by claim 1.

### EMBODIMENTS OF THE DEVICE ACCORDING TO THE INVENTION

Preferred embodiments of the invention include for example a so-called baseball-style cap, the said cap having at least one LED illuminator directed forward and inserted in the front edge of the visor of the cap, the said illuminator being an LED only or an LED provided with a reflector.

The electrical power required by the LED illuminator is obtained from thin and round button batteries, which are so light in weight and take up such a small amount of space that they can be accommodated under the cover fabric of the cap or inside the cap, for example between the cover fabric and the sweatband. Because the LED illuminator requires a supply voltage of no less than 3 V it is preferable to connect two 1.5 V button batteries in series. Rechargeable batteries can be used.

The LED illuminator used in the cap may emit white or coloured light. LED illuminators of various colours may also be used simultaneously. The forward directed illuminators of the cap may emit white light and a LED illuminator visible towards the rear may be red, for example. The illuminators may also be illuminated continuously or they may flash intermittently. A cap provided with LED illuminators also functions as an efficient means of safety as it can be easily seen in darkness. It is particularly useful as a means of safety in traffic for example for bikers and small children.

Electric wires connected between the LED illuminators and the batteries can be located under the cover fabric of the cap so as to render them invisible. The electric wires are most preferably equipped for example with a push button switch which, when pressed consecutively, first switches on the power and then switches it off.

According to one preferred embodiment of the invention, an LED illuminator attached to the visor of a cap can be tilted so that it can be directed to the required angle downwards from the horizontal level. The tilting mechanism is comprised of either a separate, tiltable plate on the lower surface of the visor or a separate piece separated from the body of the visor, to which piece the LED illuminator is attached. Between the visor and the separate plate piece, there may be a hinge or the separate plate piece itself may be made of elastic material. An elastic electrical cable connected to the LED illuminator may be used as a hinge. If the tiltable piece is a separate part separated from the body of the visor it is preferred to provide the visor with a limiter, which prevents the tiltable piece from being tilted upwards from the horizontal level.

The cap with a visor may also be provided with a pattern or a text, such as a company logo or an advertisement of a product. In such a case, an LED illuminator may be inserted in the visor, which illuminator is directed to the said pattern or text. In advertising it is important that the pattern or text to be promoted can be clearly seen. LED illuminators may also be attached to other garments, such as a blouse or the hood of a jacket.

### EMBODIMENTS

In the following, the invention is described using examples with reference to the accompanying drawings, in which

### LIST OF FIGURES

- Figure 1: represents a cap with two illuminators attached to the front edge of the visor as seen diagonally from above
- Figure 2: corresponds to Fig. 1 and represents a cap with an additional illuminator illuminating an advertisement
- Figure 3: represents a vertical sectional view of the cap in Fig. 2
- Figure 4: represents a portion of another embodiment of the cap as seen from above
- Figure 5: represents a vertical sectional view of a portion of the cap in Fig. 4
- Figure 6: represents a sectional view of the visor of the cap in Fig. 4
- Figure 7: represents a vertical sectional view of the visor of a cap according to a third embodiment
- Figure 8: corresponds to Fig. 7 and represents the illuminator attached to the visor in another position
- Figure 9: represents a cap with two illuminators and one solar cell

### DESCRIPTION OF THE FIGURES

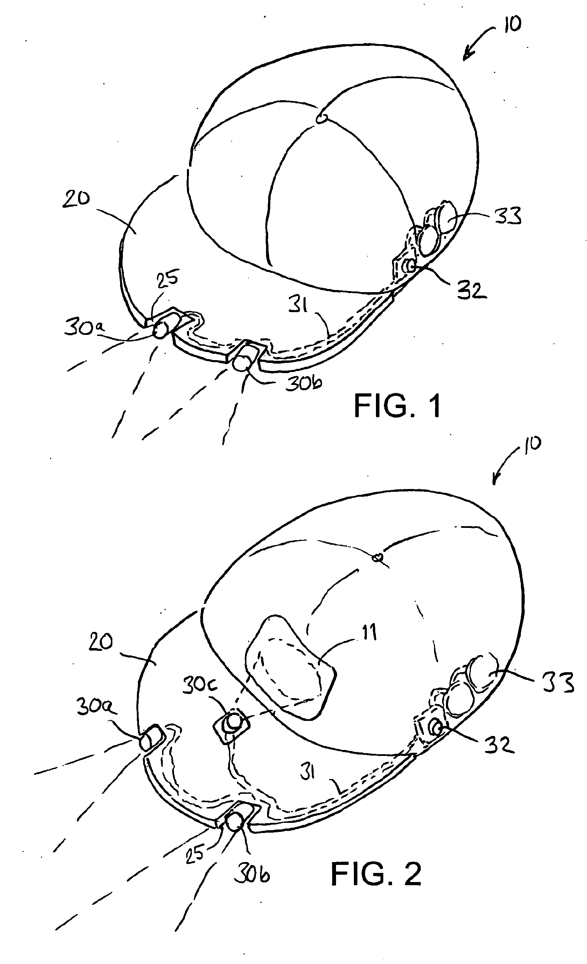

Figure 1 shows a cap 10 having two notches in the front edge of its visor 20, with forward directed LED illuminators 30a and 30b being located in the notches. Electric wires 31 of the LED illuminators 30a and 30b are connected to the side of the cap 10, to a switch 32, which connects an electric battery 33 to the LED illuminators 30a and 30b. The switch 32 and battery 33 are located inside the cap 10, between the cover fabric and the sweatband. The push button of the switch 32 is visible and comes through the cover fabric of the cap 10. The switch 32 is most preferably a push button switch of such variety that one push of the button switches on the power to the LED illuminators 30a and 30b and another push switches off the power. The LED illuminators 30a and 30b are most preferably in the notches of the plastic part of the visor 20. The fabric covering the visor covers the electric wires 31 and the LED illuminators 30a and 30b except for apertures directed forward for the lights.

Figure 2 represents two LED illuminators 30a and 30b directed forward in the visor 20 of a cap 10 and one LED illuminator 30c directed to the advertisement 11 on the front portion of the cap 10. The electric wires 31, switch 32 and batteries 33 correspond in other respects to those shown in Fig. 1. An object in front of the viewer can be seen illuminated with the help of the LED illuminators 30a and 30b of the cap 10 shown in Fig. 2. Furthermore, others can see the advertisement 11, such as a company name or a product, which is illuminated by the LED illuminator 30c, on the front portion of the cap 10. Instead of an advertisement there may be another text or piece of information, which the user wishes to highlight. Such an important message could for example be the words "Security guard", "Supervisor", "Police" or a similar message.

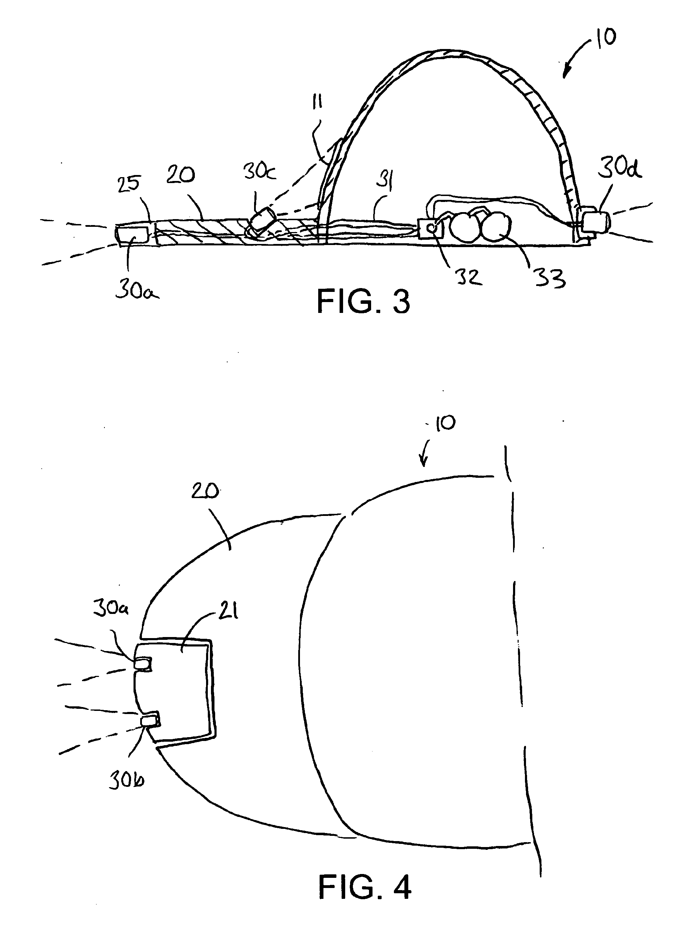

Figure 3 clearly illustrates the structure of the cap 10, where an LED illuminator 30a directed forward is located in the front edge of its visor 20, and an LED illuminator 30c directed to the advertisement 11 is inserted in the middle of the visor 20. Furthermore, there is an LED illuminator 30d directed to the back in the back portion of the cap 10. This LED illuminator 30d could for example emit red light and the other LED illuminators white light. Electric wires 31, a switch 32 and batteries 33 are located in the edge of the cap, inside of it, most preferably between the sweatband and the cover fabric of the cap 10. The plastic frame portion of the visor 20 is usually covered with fabric that should have apertures for the beams of light from the LED illuminators 30a and 30c.

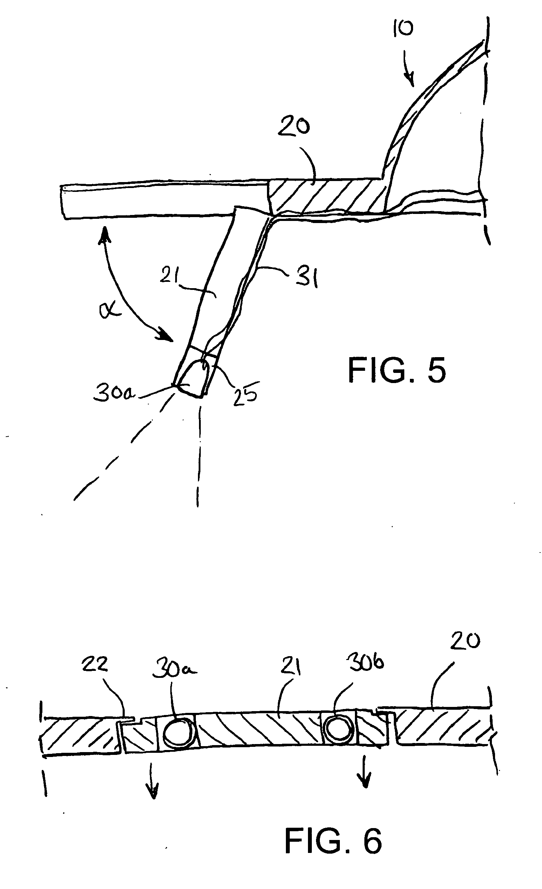

Figure 4 illustrates a cap 10 with a separate piece 21 in the visor 20. In the solution shown in Fig. 4, the separate piece 21 is cut out from the plastic frame portion of the visor 20 so that the separate piece can be moved relative to the visor 20. The LED illuminators 30a and 30b are located in the separate piece 21. The purpose of the separate piece 21 is illustrated in greater detail in the next figure, Fig. 5.

Figure 5 shows a sectional view of the visor 20 of a cap 10, the visor having a separate piece 21 shown in Fig. 4. An LED illuminator 30a is located in the front edge of the separate piece 21. Electric wires 31 are connected from the LED illuminator 30a to inside of the cap 10 so that the separate piece 21 can tilt, supported by the electric wires 31. The electric wires 31 thus function as a hinge of the separate piece 21 relative to the visor 20. With this solution, the LED illuminator 30a can be directed downwards to an object in front of the user of the cap, close to him or her. Such an object can be for example a book that the cap user is reading or the keyboard of a computer. Because the beam of light of the LED illuminator 30a is relatively narrow the beam is preferably focused only on the keyboard of a computer, for example, but not on the computer screen.

Figure 6 illustrates a cross section of the visor structure in Fig. 5, the said visor structure having the separate piece 21 of the visor 20 in its place in a gap of the visor 20. In this position, the LED illuminators 30a and 30b are directed straight forward, parallel to the visor. The separate piece 21 can, however, be tilted and the LED illuminators 30a can 30b be directed also downwards, at a required angle a relative to the horizontal level. Fig. 6 shows that rims 22 are arranged in the visor 20, which rims prevent the separate piece 21 from being tilted upwards from the level of the visor 20.

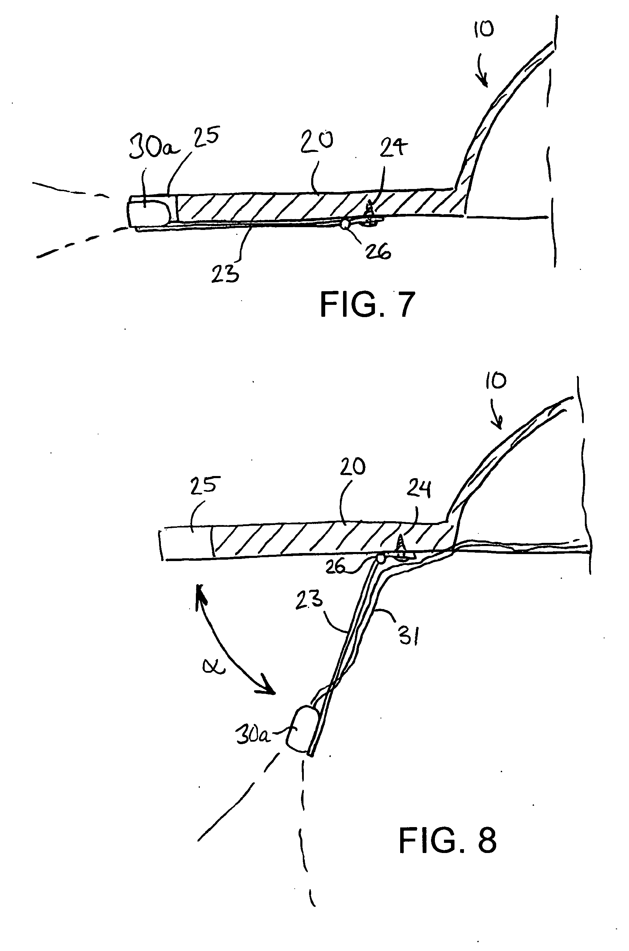

Figure 7 illustrates a solution where a plate 23 is attached with a screw 24, or alternatively with adhesive, to the lower surface of the visor 20 of a cap 10. An LED illuminator 30a is attached to the top of the front edge of the plate 23. A notch 25 is arranged on the front edge of the visor 20 so that, when the plate 23 is pressed against the visor 20, the LED illuminator 30a goes into the notch 25. There is a hinge portion 26 in the plate 23 or alternatively the plate 23 is made of an elastic material so that the plate 23 can be tilted downwards from the level of the visor 20, as presented in the next figure, Fig. 8.

Figure 8 illustrates the plate 23 attached on the lower surface of the visor 20 of the cap 10, the said plate being tilted downwards about the hinge 26. In this position, the beam of light from the LED illuminator 30 is directed downwards at a required angle α relative to the horizontal level.

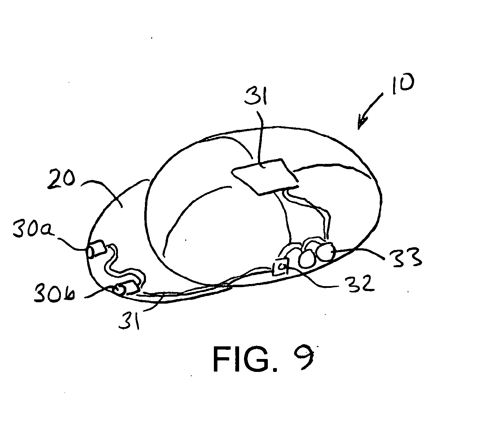

Figure 9 illustrates a cap 10 corresponding to the one in Fig. 1, which cap 10 is comprised of LED illuminators 30a and 30b, electric wires 31, a switch 32 and an electric battery 33. The electric battery 33 is a rechargeable battery in this embodiment, however, the said battery being recharged with a solar cell on top of the cap 10.

### LIST OF REVERENCE NUMBERS

- 10: Cap
- 11: Advertisement
- 20: Visor
- 21: Separate piece
- 22: Rim
- 23: Plate
- 24: Screw
- 25: Notch
- 30: LED illuminator
- 31: Wire
- 32: Switch
- 33: Electric battery
- 34: Solar cell
- 40: Hearing protector
- 41: Welding mask
- 42: Bike helmet
- 43: Diving mask
- 44: Sweatband
- 45: Billboard
- 46: Wrist illuminator
- 47: Walking pole
- 48: Handle
- 49: Electric battery
- 50: Light switch
- 51: Car
- 52: Cord of a heater
- 53: Pole
- 54: Receptacle
- 55: Receptacle of a car

## Claims

1. A cap (10) comprising a cover fabric, to which at least one small-sized LED illuminator (30) emitting white light and a power supply required by the illuminator, such as a rechargeable or a disposable battery, and a switch (32) is attached, at least one LED illuminator (30a) emitting white light is located substantially inside a visor (20) of the cap, **characterised in that** the power supply comprises an arrangement of two button batteries, which are connected in series and located adjacent to each other in a side by side arrangement, and being arranged in the direction of and along the base circumference of the cap, under the cover fabric of the cap.

2. A cap (10, 42) as claimed in claim 1, **characterised in that**
- the frame portion of the visor (20) of the cap, is covered with fabric so that the LED illuminator is in a notch (25) of the frame portion of the visor (20), inside the fabric, and
- furthermore, there is an aperture in the fabric for the beam of light from the LED illuminator (30a).

3. A cap (10, 42) as claimed in claim 1 or 2, **characterised in that** there is at least one LED illuminator (30a) emitting white light in the front edge of the visor (20) of the cap, which LED illuminator is most preferably directed substantially forward.

4. A cap (10, 42) as claimed in claim 1, 2 or 3, **characterised in that** there is at least one LED illuminators (30a) emitting white light in the front edge of the visor (20) of the cap, which LED illuminator can be tilted downwards to a required angle (α) so that the LED illuminator can be used as a spot light to facilitate the work being performed.

5. A cap (10, 42) as claimed in any of the claims 1-4, **characterised in that** there is at least one LED Illuminator (30c) emitting white light in the visor (20) of the cap, which LED illuminator is directed to an object attached to the cap, such as an advertisement (11).

6. A cap (10, 42) as claimed in any of the claims 1-5, **characterised in that** there is at least one coloured, rear-facing light, such as an LED illuminator (30d) emitting red light, the said light being in the back portion of the cap.

7. A cap (10, 42) as claimed in any of the claims1-6, **characterised in that** there are two or more LED illuminators (30a, 30b) directed forward in the visor (20) of the cap.

8. A cap (10, 42) as claimed in any of the claims 1-7, **characterised in that** the LED illuminator (30a) in the visor (20) of the cap is provided with a reflector to increase the lighting power.

9. A cap (10, 42) as claimed in any of the claims 1-8, **characterised in that** at least one LED illuminator (30) in the cap flashes intermittently.

## Patentansprüche

1. Eine einen Bezugsstoff enthaltende Kappe (10), an welcher wenigstens eine kleine weißes Licht emittierende LED-Leuchte (30) und eine von der Leuchte benötigte Stromversorgung, wie z.B. eine wiederaufladbare oder Einweg-Batterie und ein Schalter (32) angebracht sind, wobei wenigstens eine weißes Licht emittierende LED-Leuchte (30a) im Wesentlichen innerhalb eines Schirms (20) der Kappe angeordnet ist, **dadurch gekennzeichnet, dass** die Stromversorgung eine Anordnung von zwei Knopfbatterien enthält, die in Serie geschaltet und seitlich nebeneinander und in Richtung und entlang des Basisumfangs der Kappe unter dem Bezugsstoff der Kappe angeordnet sind.

2. Eine Kappe (10, 42) nach Anspruch 1, **dadurch gekennzeichnet, dass** der Rahmenteil des Schirms (20) der Kappe mit einem Stoff bezogen ist, so dass die LED-Leuchte in einer Aussparung (25) des Rahmenteils des Schirms (20) innerhalb des Stoffes angeordnet ist, und dass weiterhin eine Öffnung in dem Stoff vorhanden ist für den Lichtstrahl von der LED-Leuchte (30a).

3. Kappe (10, 42) nach Anspruch 1 oder 2, **dadurch gekennzeichnet, dass** wenigstens eine weißes Licht emittierende LED-Leuchte (30a) in der Vorderkante des Schirms (20) der Kappe vorhanden ist, welche LED-Leuchte vorzugsweise im Wesentlichen nach vorne ausgerichtet ist.

4. Kappe (10, 42) nach Anspruch 1, 2 oder 3, **dadurch gekennzeichnet, dass** wenigstens eine weißes Licht emittierende LED-Leuchte (30a) in der Vorderkante des Schirms (20) der Kappe vorgesehen ist, welche LED-Leuchte nach unten in einem erforderlichen Winkel (α) geneigt werden kann, so dass die LED-Leuchte als Punkte-Leuchte zum Erleichtern einer auszuführenden Arbeit verwendet werden kann.

5. Kappe (10, 42) nach einem der Ansprüche 1 - 4, **dadurch gekennzeichnet, dass** wenigstens eine weißes Licht emittierende LED-Leuchte (30c) in dem Schirm (20) der Kappe vorgesehen ist, welche LED-Leuchte direkt auf ein an der Kappe befestigtes Objekt gerichtet ist, wie z.B. ein Logo bzw. eine Werbeanzeige (11).

6. Kappe (10, 42) nach einem der Ansprüche 1 - 5, **dadurch gekennzeichnet, dass** wenigstens ein farbiges nach hinten weisendes Licht vorgesehen ist, wie z.B. eine rotes Licht emittierende LED-Leuchte (30d), welches Licht in dem hinteren Abschnitt der Kappe angeordnet ist.

7. Kappe (10, 42) nach einem der Ansprüche 1 - 6, **dadurch gekennzeichnet, dass** in dem Schirm (20) der Kappe zwei oder mehrere nach vorne gerichtete LED-Leuchten (30a, 30b) vorgesehen sind.

8. Kappe (10, 42) nach einem der Ansprüche 1 - 7, **dadurch gekenntzeichnet, dass** die LED-Leuchte (30a) in dem Schirm (20) der Kappe mit einem Reflektor versehen ist, um die Lichtstärke zu erhöhen.

9. Kappe (10, 42) nach einem der Ansprüche 1 - 8, **dadurch gekennzeichnet, dass** wenigstens eine LED-Leuchte (30) in der Kappe blinkt bzw. periodisch aufblitzt.

## Revendications

1. Casquette (10) comprenant un tissu de recouvrement, à laquelle au moins un dispositif d'éclairage à DEL (30) de petite taille émettant une lumière blanche et une alimentation nécessaire au dispositif d'éclairage, telle qu'une batterie rechargeable ou jetable, et un commutateur (32) sont fixés, au moins un dispositif d'éclairage à DEL (30a) émettant une lumière blanche étant situé sensiblement à l'intérieur d'une visière (20) de la casquette,
**caractérisée en ce que** l'alimentation comprend un agencement de deux batteries boutons, qui sont connectées en série et situées adjacentes l'une à l'autre en un agencement côte à côte, et qui sont agencées dans la direction et le long de la circonférence de base de la casquette, sous le tissu de recouvrement de la casquette.

2. Casquette (10, 42) selon la revendication 1, **caractérisée en ce que**
- la partie de cadre de la visière (20) de la casquette est recouverte de tissu de sorte que le dispositif d'éclairage à DEL se trouve dans une encoche (25) de la partie de cadre de la visière (20), à l'intérieur du tissu, et
- en outre, il y a une ouverture dans le tissu pour le faisceau de lumière provenant du dispositif d'éclairage à DEL (30a).

3. Casquette (10, 42) selon la revendication 1 ou 2, **caractérisée en ce qu'**il y a au moins un dispositif d'éclairage à DEL (30a) émettant une lumière blanche dans le bord avant de la visière (20) de la casquette, lequel dispositif d'éclairage à DEL est, de manière plus préférentielle, dirigé sensiblement vers l'avant.

4. Casquette (10, 42) selon la revendication 1, 2 ou 3, **caractérisée en ce qu'**il y a au moins un dispositif d'éclairage à DEL (30a) émettant une lumière blanche dans le bord avant de la visière (20) de la casquette, lequel dispositif d'éclairage à DEL peut être incliné vers le bas selon un angle (α) requis de sorte que le dispositif d'éclairage à DEL peut être utilisé en tant qu'éclairage directionnel pour faciliter la tâche effectuée.

5. Casquette (10, 42) selon l'une quelconque des revendications 1 à 4, **caractérisée en ce qu'**il y a au moins un dispositif d'éclairage à DEL (30c) émettant une lumière blanche dans la visière (20) de la casquette, lequel dispositif d'éclairage à DEL est dirigé vers un objet attaché à la casquette, tel qu'une publicité (11).

6. Casquette (10, 42) selon l'une quelconque des revendications 1 à 5, **caractérisée en ce qu'**il y a au moins une lampe colorée orientée vers l'arrière, telle qu'un dispositif d'éclairage à DEL (30d) émettant une lumière rouge, ladite lampe étant dans la partie arrière de la casquette.

7. Casquette (10, 42) selon l'une quelconque des revendications 1 à 6, **caractérisée en ce qu'**il y a au moins deux dispositifs d'éclairage à DEL (30a, 30b) ou plus dirigés vers l'avant dans la visière (20) de la casquette.

8. Casquette (10, 42) selon l'une quelconque des revendications 1 à 7, **caractérisée en ce que** le dispositif d'éclairage à DEL (30a) dans la visière (20) de la casquette est pourvu d'un réflecteur pour augmenter la puissance lumineuse.

9. Casquette (10, 42) selon l'une quelconque des revendications 1 à 8, **caractérisée en ce qu'**au moins un dispositif d'éclairage à DEL (30) dans la casquette clignote de manière intermittente.
